# EUROPEAN PATENT APPLICATION

(11) **EP 3 881 955 A1**
(43) Date of publication of application: **22.09.2021**
(21) Application number: 20164572.8
(22) Date of filing: 20.03.2020
(51) Int. Cl.: B22F 1/00, B22F 9/24, B01J 23/44, B01J 35/00, B01J 37/00

(54) **PROCESS FOR THE PREPARATION OF TRANSITION METAL NANOPARTICLES**

(71) Applicant: BASF Corporation, Florham Park, NJ 07932 (US)
(72) Inventor: IACONO, Giovanni, 00131 Roma (IT); RISICA, Daniela, 00131 Roma (IT)
(74) Representative: Altmann Stößel Dick Patentanwälte PartG mbB

(57) **Abstract**

A process for the preparation of transition metal nanoparticles, the process comprising:
(a) providing a mixture of one or more salts of one or more transition metals M, one or more complexing agents C, and a solvent system S;
(b) optionally adjusting the pH of the mixture provided in (a) to a pH comprised in the range of from 4 to 8;
(c) heating the mixture provided in (a) or obtained in (b) for obtaining a colloidal suspension of transition metal nanoparticles;
(d) optionally isolating the transition metal nanoparticles obtained in (c), preferably by centrifugation and/or evaporation to dryness of the colloidal suspension obtained in (c)
wherein the mixture provided in (a) and heated in (c) or obtained in (b) and heated in (c) does not comprise polyvinyl sulfate and/or polyvinylpyrrolidone.

## Description

### TECHNICAL FIELD

The present invention relates to a process for preparing transition metal nanoparticles, a colloidal suspension of transition metal nanoparticles, transition metal nanoparticles and the use thereof as a catalyst.

### INTRODUCTION

Colloidal palladium is typically prepared by using two reagents, in particular a suspending agent and a reducing agent, usually polyvinylpyrrolidone as suspending agent. As far as a reducing agent is concerned, one or more of ascorbic acid, sodium borohydride, citric acid, and formic acid/formate are typically used.

Generally, several different recipes can be found in the prior art which are however limited to low palladium concentrations, in particular smaller than 0.1 weight-%. Another constriction is caused by steric effects of a suspending agent hindering diffusion of an organic substrate in the colloidal palladium, when deposited on a porous support.

US 10,493,433 B2 relates to colloidal precious metal nanoparticles with high precious metal concentration for catalyst applications and methods for their synthesis. Further, a colloidal dispersion is disclosed therein comprising a dispersion medium, a water-soluble polymer suspension stabilizing agent, and a reducing agent. The precious metal is selected from one or more of Pt, Pd, Au, Ag, Ru, Rh, Ir, and Os. Z.-L. Wang et al disclose in Scientific reports a study on Pd nanoparticles on carbon synthesized in the presence of citric acid. M. Michaelis et al. disclose in J. Phys. Chem. preparation of a Pd colloid stabilized with polyvinylsulfate wherein citrate is used to reduce Pd(II). H. Lv et al. disclose in Nano Energy a study on the design of tailored nano-materials for efficient oxygen reduction reaction. In particular, preparation of nanoparticles is disclosed using sodium tetrachloropalladate, citric acid, ethanol, and polyvinylpyrrolidone. M. Shao et al. disclose in Chem. Commun. a synthesis of Pd octahedra using polyvinylpyrrolidone, citric acid, and sodium tetrachloropalladate.

Therefore, it was an object to provide an improved process for preparing colloidal suspensions and nanoparticles of one or more transition metals, in particular a process not suffering from the above mentioned drawbacks but allowing an economical advantageous process and especially allowing use of comparatively high concentrations of a transition metal. Further, it was an object to provide transition metal nanoparticles and a suspension of a colloid of transition metal nanoparticles.

Surprisingly, it was found that a novel process for the preparation of transition metal nanoparticles can be provided, the process particularly comprising providing a mixture of one or more salts of one or more transition metals M, one or more complexing agents C, and a solvent system S, optionally adjusting the pH of said mixture to a pH comprised in the range of from 4 to 8, and heating the obtained mixture for obtaining a colloidal suspension of transition metal nanoparticles, wherein the mixture does not comprise polyvinyl sulfate and/or polyvinylpyrrolidone.

Further, it was found that a novel process for the preparation of transition metal nanoparticles can be provided, the process preferably using only one reagent, wherein the reagent preferably has a low steric hindrance, and which can act as suspending agent and as reducing agent. In particular, one or more complexing agents can be used exhibiting a comparatively low steric hindrance, being able to form a colloid with a flexible structure, and which is able to generate no diffusional control or mass transport restriction to the one or more complexing agents to be bonded to a transition metal and hydrogenated.

Further, it was surprisingly found that the novel process for the preparation of transition metal nanoparticles according to the present invention allows for a comparatively high transition metal concentration of a colloidal suspension. In addition thereto, it was found that transition metal nanoparticles can be provided having an average particle size of up to 2 nm.

Therefore, the present invention relates to a process for the preparation of transition metal nanoparticles, the process comprising:
(a) providing a mixture of one or more salts of one or more transition metals M, one or more complexing agents C, and a solvent system S;
(b) optionally adjusting the pH of the mixture provided in (a) to a pH comprised in the range of from 4 to 8;
(c) heating the mixture provided in (a) or obtained in (b) for obtaining a colloidal suspension of transition metal nanoparticles;
wherein the mixture provided in (a) and heated in (c) or obtained in (b) and heated in (c) does not comprise polyvinyl sulfate and/or polyvinylpyrrolidone.

It is preferred that the mixture provided in (a) and heated in (c) or obtained in (b) and heated in (c) does not comprise a polyvinyl sulfate, polyvinylpyrrolidone, a copolymer comprising vinylpyrrolidone, and/or a fatty acid-substituted or unsubstituted polyoxyethylene, more preferably does not comprise a polyvinyl and/or a polyoxyethylene compound, more preferably does not comprise a polymer compound, more preferably does not comprise a complexing agent other than the one or more complexing agents C.

It is preferred that the mixture provided in (a) and heated in (c) or obtained in (b) and heated in (c) does not comprise ascorbic acid, an ascorbate, formic acid, a formate, and/or a borohydride, wherein more preferably the mixture provided in (a) and heated in (c) or obtained in (b) and heated in (c) does not comprise ascorbic acid, an ascorbate, formic acid, a formate, a borohydride, hydrogen, hydrazine, hydroxyethylhydrazine, formic hydrazide, urea, formaldehyde, glucose, sucrose, xylitol, meso-erythritol, sorbitol, glycerol, maltitol, oxalic acid, methanol, ethanol, 1-propanol, iso-propanol, 1-butanol, 2-butanol, 2-methyl-propan-1-ol, allyl alcohol, diacetone alcohol, ethylene glycol, propylene glycol, diethylene glycol, tetraethylene glycol, dipropylene glycol, tannic acid, a tannate, gallic acid, and/or a gallate, wherein more preferably the mixture provided in (a) and heated in (c) or obtained in (b) and heated in (c) does not comprise a reducing agent other than the one or more complexing agents C.

It is preferred that the mixture provided in (a) and heated in (c) or obtained in (b) and heated in (c) consists of the mixture of the one or more salts of one or more transition metals M, the one or more complexing agents C, the solvent system, and optionally one or more compounds employed for adjusting the pH of the mixture in (b).

It is preferred that the one or more complexing agents C are selected from the group consisting of carboxylates and salts thereof,
preferably from the group consisting of optionally protonated monocarboxylates, dicarboxylates, tricarboxylates, tetracarboxylates, and polycarboxylates, including mixtures of two or more thereof,
more preferably from the group consisting of optionally protonated dicarboxylates, tricarboxylates, and tetracarboxylates, including mixtures of two or more thereof,
more preferably from the group consisting of optionally protonated oxalates, malonates, succinates, glutarates, adipates, pimelates, suberates, azelaiates, sebacates, undecanoates, dodecanoates, citrates, isocitrates, aconitates, propane-1,2,3-tricarboxylates, trimesates, furantetracarboxylates, methanetetracarboxylates, and ethylenetetracarboxylates, including mixtures of two or more thereof,
more preferably from the group consisting of optionally protonated malonates, succinates, glutarates, adipates, citrates, isocitrates, aconitates, propane-1,2,3-tricarboxylates, methanetetracarboxylates, and ethylenetetracarboxylates, including mixtures of two or more thereof,
more preferably from the group consisting of optionally protonated succinates, glutarates, citrates, isocitrates, propane-1,2,3-tricarboxylates, and methanetetracarboxylates, including mixtures of two or more thereof,
more preferably from the group consisting of optionally protonated succinates, citrates, isocitrates, and propane-1,2,3-tricarboxylates, including mixtures of two or more thereof,
more preferably from the group consisting of optionally protonated citrates and isocitrates, including mixtures of two or more thereof,
wherein more preferably the one or more complexing agents C comprise optionally protonated citrates, including mixtures of two or more thereof, preferably citrates, including mixtures of two or more thereof
wherein more preferably the one or more complexing agents C consist if optionally protonated citrates, including mixtures of two or more thereof, preferably of citrates, including mixtures of two or more thereof,
wherein the counterion of the unprotonated form or forms of the one or more complexing agents C is selected from the group consisting of alkali metals, alkaline earth metals, ammonium, and combinations of two or more thereof, wherein preferably the counterion is Na⁺ and/or K⁺, preferably Na⁺.

It is preferred that the mixture provided in (a) and heated in (c) or obtained in (b) and heated in (c) does not comprise a complexing agent other than the one or more complexing agents C.

It is preferred that in (b) the pH of the mixture provided in (a) is adjusted to a pH comprised in the range of from 5 to 7, more preferably from 5.5 to 6.5, more preferably from 5.9 to 6.3, more preferably from 6.0 to 6.2, and more preferably to about a pH of 6.1.

It is preferred that in (a) the one or more transition metals M are selected from the group consisting of Cu, Ru, Rh, Pd, Ag, Re, Os, Ir, Pt, and Au, including mixtures of two or more thereof, more preferably from the group consisting of Pd, Pt, Ru, Rh, Au, and Ag, including mixtures of two or more thereof, more preferably from the group consisting of Pd, Pt, Au, and Ag, including mixtures of two or more thereof, wherein more preferably the one or more transition metals M comprise Pd and/or Pt, preferably Pd, wherein more preferably the one or more transition metals M consist of Pd and/or Pt, preferably of Pd.

It is preferred that in (a) the counterion of the one or more salts of one or more transition metals M is selected from the group consisting of halides, hydroxide, nitrate, phosphate, sulfate, and combinations of two or more thereof, more preferably from the group consisting of chloride, bromide, hydroxide, nitrate, sulfate, and combinations of two or more thereof, wherein more preferably the counterion is chloride and/or nitrate, preferably chloride.

It is preferred that in (a) the one or more salts of the one or more transition metals M are provided as a halide complex, more preferably as a chloride complex, and more preferably as a tetrachloro complex, wherein the counterion of the complex is preferably selected from the group consisting of H⁺, alkali metals, alkaline earth metals, ammonium, and combinations of two or more thereof, more preferably from the group consisting of Na⁺, K⁺, H⁺, and combinations of two or more thereof, wherein more preferably the counterion is Na⁺ and/or H⁺, preferably Na⁺.

It is preferred that in the mixture provided in (a), the C : M molar ratio of the one or more complexing agents C to the one or more transition metals M is comprised in the range of from 1 to 200, more preferably from 5 to 100, more preferably from 10 to 50, more preferably from 15 to 40, more preferably from 20 to 30, more preferably from 23 to 27, and more preferably from 25 to 26.

It is preferred that the solvent system S employed in (a) comprises one or more polar solvents, more preferably one or more polar protic solvents, more preferably one or more polar protic solvents selected from the group consisting of C1-C4 alcohols, water, dimethylformamide, and mixtures of two or more thereof, more preferably from the group consisting of n-propanol, iso-propanol, methanol, ethanol, water, and mixtures of two or more thereof, more preferably from the group consisting of methanol, ethanol, dimethylformamide, water, and mixtures of two or more thereof, wherein more preferably the solvent system comprises ethanol and/or water, preferably water, wherein more preferably the solvent system consists of ethanol and/or water, preferably of water.

It is preferred that in the mixture provided in (a), the S : C molar ratio of the solvent system S to the one or more complexing agents C is comprised in the range of from 1 to 200, more preferably from 5 to 100, more preferably from 10 to 50, more preferably from 15 to 40, more preferably from 20 to 30, and more preferably from 24.2 to 26.2.

It is preferred that in the mixture provided in (a), the S : M molar ratio of the solvent system S to the one or more transition metals M is comprised in the range of from 50 to 3500, more preferably from 60 to 2000, more preferably from 75 to 1000, more preferably from 300 to 900, preferably from 400 to 800, preferably from 500 to 700, more preferably from 620 to 665, and more preferably from 642 to 643.

It is preferred that in (b) the pH of the mixture provided in (a) is adjusted using a base, more preferably using a salt of a hydroxide, more preferably using an alkali metal hydroxide, wherein the alkali metal is preferably selected from the group consisting of Na, K, Rb, and Cs, wherein more preferably the alkali metal hydroxide is sodium hydroxide and/or potassium hydroxide, wherein more preferably the alkali metal hydroxide is sodium hydroxide.

It is preferred that in (b) the pH of the mixture provided in (a) is adjusted using an acid, more preferably using a mineral acid, more preferably using a mineral acid selected from the group consisting of HCl, HBr, HI, HClO, HClO₂, HClO₃, HClO₄, H₂SO₄, HSO₃F, HNO₃, H₃PO₄, HSBF₆, HBF₄, and HPF₆, including mixtures of two or more thereof, more preferably from the group consisting of HCl, HBr, HClO₄, H₂SO₄, HSO₃F, HNO₃, and H₃PO₄, including mixtures of two or more thereof, more preferably from the group consisting of HCl, HBr, H₂SO₄, HNO₃, and H₃PO₄, including mixtures of two or more thereof, more preferably from the group consisting of HCl, H₂SO₄, and HNO₃, including mixtures of two or more thereof, wherein more preferably the mixture provided in (a) is adjusted using HCl.

In the case where in (b) the pH of the mixture provided in (a) is adjusted using an acid, it is preferred that the acid is employed as an aqueous solution, more preferably as an aqueous solution comprising from 5 to 60 wt.-% of the acid, more preferably from 10 to 50 wt.-%, more preferably from 20 to 40 wt.-%, more preferably from 25 to 39 wt.-%, more preferably from 29 to 37 wt.-%, more preferably from 31 to 35 wt.-%, and more preferably from 32 to 34 wt.-%.

It is further that in (c) the mixture provided in (a) or obtained in (b) is heated to a temperature comprised in the range of from 80 to 120 °C, more preferably from 90 to 110 °C, more preferably from 95 to 105 °C, more preferably from 98 to 102 °C, and more preferably from 99 to 101 °C.

It is preferred that in (c) the mixture provided in (a) or obtained in (b) is heated to a temperature comprised in the range of from 70 °C to the boiling point of the mixture provided in (a) or obtained in (b), more preferably from 90 °C to the boiling point of the mixture provided in (a) or obtained in (b), wherein more preferably the mixture provided in (a) or obtained in (b) is heated to the boiling point of the mixture provided in (a) or obtained in (b), wherein the boiling point refers to the boiling temperature at 100 kPa.

It is preferred that in (c) the mixture provided in (a) or obtained in (b) is heated for a duration comprised in the range of from 1 to 360 minutes, more preferably from 5 to 60 minutes, more preferably from 10 to 40 minutes, more preferably from 10 to 20 minutes, more preferably from 12 to 18 minutes, and more preferably about 15 minutes.

It is preferred that in (c) the mixture provided in (a) or obtained in (b) is heated under a pressure comprised in the range of from 90 to 110 kPa, more preferably of from 95 to 105 kPa, more preferably of from 98 to 102 kPa, and more preferably from 99 to 101 kPa, wherein more preferably the mixture provided in (a) or obtained in (b) is heated under atmospheric pressure.

It is preferred that the process further comprises:
(e) contacting the colloidal suspension of transition metal nanoparticles obtained in (c) or the transition metal nanoparticles obtained in (d) with a support material for supporting the transition metal nanoparticles on the support material.

In the case where the process further comprises (e), it is preferred that the support material in (e) comprises carbon and/or a metal oxide and/or a metalloid oxide, more preferably activated carbon and/or an oxide selected from the group consisting of oxides of Si, Al, Ti, Zr, Hf, La, Ce, Pr, Nd, and mixtures and/or mixed oxides of two or more thereof, more preferably from the group consisting of oxides of Si, Al, Ti, Zr, and mixtures and/or mixed oxides of two or more thereof, wherein more preferably the support material is selected from the group consisting of activated carbon, silica, alumina, silica-alumina, aluminosilicates, titanosilicates, and mixtures of two or more thereof, wherein more preferably the support material comprises activated carbon and/or silicalite, preferably activated carbon, wherein more preferably the support material consists of activated carbon and/or silicalite, preferably of activated carbon.

Further in the case where the process further comprises (e), it is preferred that the support material in (e) is a monolith substrate and/or is in the form of granules and/or is in the form of a powder.

Further in the case where the process further comprises (e), it is preferred that contacting in (e) is achieved by impregnation, more preferably by incipient wetness impregnation and/or by vacuum impregnation.

Furthermore, the present invention relates to a colloidal suspension of transition metal nanoparticles obtainable and/or obtained according to the process of any one of the embodiments disclosed herein.

It is preferred that the concentration of the transition metal nanoparticles in the colloidal suspension is comprised in the range of from 0.01 to 5 wt.-%, more preferably from 0.1 to 4 wt.-%, more preferably from 0.2 to 3 wt.-%, more preferably from 0.4 to 2 wt.-% and more preferably from 0.5 to 1 wt.-%, wherein the total weight of the transition metal nanoparticles corresponds to the total weight of the transition metal contained in the colloidal suspension calculated as the element.

It is preferred that the weight-based average particle size D50 of the transition metal nanoparticles is in the range of from 0.2 to 20 nm, more preferably from 0.4 to 10 nm, more preferably from 0.6 to 5 nm, more preferably from 0.8 to 4 nm, more preferably from 1 to 3, nm more preferably from 1.2 to 2.5 nm, more preferably from 1.4 to 2.3 nm, more preferably from 1.6 to 2.1 nm, more preferably from 1.65 to 1.9 nm, and more preferably from 1.7 to 1.8 nm, wherein preferably the particle size distribution is determined according to ISO 13320-1, and the weight-based average particle size D50 is calculated based thereon according to ISO 9276-2:2001.

It is preferred that the molecular weight of the transition metal nanoparticles is in the range of from 3000 to 50000 Dalton, preferably in the range of from 4000 to 10000 Dalton, more preferably in the range of from 5000 to 8000 Dalton, preferably determined according to Reference Example 2.

It is preferred that the transition metal of the nanoparticles is selected from the group consisting of Cu, Ru, Rh, Pd, Ag, Re, Os, Ir, Pt, Au, and alloys of two or more thereof, more preferably from the group consisting of Pd, Pt, Ru, Rh, Au, Ag, and alloys of two or more thereof, and more preferably from the group consisting of Pd, Pt, Au, Ag, and alloys of two or more thereof, wherein more preferably the transition metal of the nanoparticles comprises Pd, Pt, PdAu, PdAg, PtAu, or PtAg, preferably Pd, PdAg, or PdAu, more preferably Pd or PdAu, and more preferably Pd, wherein more preferably the transition metal of the nanoparticles is Pd, Pt, PdAu, PdAg, PtAu, or PtAg, preferably Pd, PdAg, or PdAu, more preferably Pd or PdAu, and more preferably Pd.

Yet further, the present invention relates to transition metal nanoparticles obtainable and/or obtained according to the process of any one of the embodiments disclosed herein, in particular according to the embodiments for the process for the preparation of transition metal nanoparticles as disclosed herein and comprising (d).

It is preferred that the weight-based average particle size D50 of the transition metal nanoparticles is in the range of from 0.2 to 20 nm, more preferably from 0.4 to 10 nm, more preferably from 0.6 to 5 nm, more preferably from 0.8 to 4 nm, more preferably from 1 to 3, nm more preferably from 1.2 to 2.5 nm, more preferably from 1.4 to 2.3 nm, more preferably from 1.6 to 2.1 nm, more preferably from 1.65 to 1.9 nm, and more preferably from 1.7 to 1.8 nm, wherein preferably the particle size distribution is determined according to ISO 13320-1, and the weight-based average particle size D50 is calculated based thereon according to ISO 9276-2:2001.

It is preferred that the molecular weight of the transition metal nanoparticles is in the range of from 3000 to 50000 Dalton, more preferably in the range of from 4000 to 10000 Dalton, more preferably in the range of from 5000 to 8000 Dalton, preferably determined according to Reference Example 2.

It is preferred that the transition metal of the nanoparticles is selected from the group consisting of Cu, Ru, Rh, Pd, Ag, Re, Os, Ir, Pt, Au, and alloys of two or more thereof, more preferably from the group consisting of Pd, Pt, Ru, Rh, Au, Ag, and alloys of two or more thereof, and more preferably from the group consisting of Pd, Pt, Au, Ag, and alloys of two or more thereof, wherein more preferably the transition metal of the nanoparticles comprises Pd, Pt, PdAu, PdAg, PtAu, or PtAg, preferably Pd, PdAg, or PdAu, more preferably Pd or PdAu, and more preferably Pd, wherein more preferably the transition metal of the nanoparticles is Pd, Pt, PdAu, PdAg, PtAu, or PtAg, preferably Pd, PdAg, or PdAu, more preferably Pd or PdAu, and more preferably Pd.

Furthermore, the present invention relates to a catalyst comprising transition metal nanoparticles obtainable and/or obtained according to the process of any one of the embodiments disclosed herein, in particular according to the embodiments for the process for the preparation of transition metal nanoparticles as disclosed herein and comprising (e).

It is preferred that the catalyst comprises from 0.01 to 10 wt.-% of transition metal nanoparticles calculated as the transition metal and based on 100 wt.-% of the support material, more preferably from 0.05 to 6 wt.-%, more preferably from 0.1 to 4 wt.-%, more preferably from 0.3 to 3 wt.-%, more preferably from 0.5 to 2.5 wt.-%, more preferably from 0.6 to 2 wt.-%, more preferably from 0.7 to 1.5 wt.-%, more preferably from 0.8 to 1.3 wt.-%, and more preferably from 0.9 to 1.1 wt.-%.

Furthermore, the present invention relates to a use of a catalyst according to any one of the embodiments disclosed herein as a hydrogenation catalyst, preferably as a hydrogenation catalyst in the production of hydrogen peroxide, and more preferably as a hydrogenation catalyst in the anthraquinone process for the production of hydrogen peroxide.

The unit bar(abs) refers to an absolute pressure of 10⁵ Pa and the unit Angstrom refers to a length of 10⁻¹⁰ m.

The present invention is further illustrated by the following set of embodiments and combinations of embodiments resulting from the dependencies and back-references as indicated . In particular, it is noted that in each instance where a range of embodiments is mentioned, for example in the context of a term such as "any one of embodiments (1) to (4)", every embodiment in this range is meant to be explicitly disclosed for the skilled person, i.e. the wording of this term is to be understood by the skilled person as being synonymous to "any one of embodiments (1), (2), (3), and (4)". Further, it is explicitly noted that the following set of embodiments is not the set of claims determining the extent of protection, but represents a suitably structured part of the description directed to general and preferred aspects of the present invention.

According to an embodiment (1), the present invention relates to a process for the preparation of transition metal nanoparticles, the process comprising:
(a) providing a mixture of one or more salts of one or more transition metals M, one or more complexing agents C, and a solvent system S;
(b) optionally adjusting the pH of the mixture provided in (a) to a pH comprised in the range of from 4 to 8;
(c) heating the mixture provided in (a) or obtained in (b) for obtaining a colloidal suspension of transition metal nanoparticles;
(d) optionally isolating the transition metal nanoparticles obtained in (c), preferably by centrifugation and/or evaporation to dryness of the colloidal suspension obtained in (c);
wherein the mixture provided in (a) and heated in (c) or obtained in (b) and heated in (c) does not comprise polyvinyl sulfate and/or polyvinylpyrrolidone.

A preferred embodiment (2) concretizing embodiment (1) relates to said process, wherein the mixture provided in (a) and heated in (c) or obtained in (b) and heated in (c) does not comprise a polyvinyl sulfate, polyvinylpyrrolidone, a copolymer comprising vinylpyrrolidone, and/or a fatty acid-substituted or unsubstituted polyoxyethylene, preferably does not comprise a polyvinyl and/or a polyoxyethylene compound, more preferably does not comprise a polymer compound, more preferably does not comprise a complexing agent other than the one or more complexing agents C.

A further preferred embodiment (3) concretizing embodiment (1) or (2) relates to said process, wherein the mixture provided in (a) and heated in (c) or obtained in (b) and heated in (c) does not comprise ascorbic acid, an ascorbate, formic acid, a formate, and/or a borohydride, wherein preferably the mixture provided in (a) and heated in (c) or obtained in (b) and heated in (c) does not comprise ascorbic acid, an ascorbate, formic acid, a formate, a borohydride, hydrogen, hydrazine, hydroxyethylhydrazine, formic hydrazide, urea, formaldehyde, glucose, sucrose, xylitol, meso-erythritol, sorbitol, glycerol, maltitol, oxalic acid, methanol, ethanol, 1-propanol, iso-propanol, 1-butanol, 2-butanol, 2-methyl-propan-1-ol, allyl alcohol, diacetone alcohol, ethylene glycol, propylene glycol, diethylene glycol, tetraethylene glycol, dipropylene glycol, tannic acid, a tannate, gallic acid, and/or a gallate, wherein more preferably the mixture provided in (a) and heated in (c) or obtained in (b) and heated in (c) does not comprise a reducing agent other than the one or more complexing agents C.

A further preferred embodiment (4) concretizing any one of embodiments (1) to (3) relates to said process, wherein the mixture provided in (a) and heated in (c) or obtained in (b) and heated in (c) consists of the mixture of the one or more salts of one or more transition metals M, the one or more complexing agents C, the solvent system, and optionally one or more compounds employed for adjusting the pH of the mixture in (b).

A further preferred embodiment (5) concretizing any one of embodiments (1) to (4) relates to said process, wherein the one or more complexing agents C are selected from the group consisting of carboxylates and salts thereof, preferably from the group consisting of optionally protonated monocarboxylates, dicarboxylates, tricarboxylates, tetracarboxylates, and polycarboxylates, including mixtures of two or more thereof,
more preferably from the group consisting of optionally protonated dicarboxylates, tricarboxylates, and tetracarboxylates, including mixtures of two or more thereof,
more preferably from the group consisting of optionally protonated oxalates, malonates, succinates, glutarates, adipates, pimelates, suberates, azelaiates, sebacates, undecanoates, dodecanoates, citrates, isocitrates, aconitates, propane-1,2,3-tricarboxylates, trimesates, furantetracarboxylates, methanetetracarboxylates, and ethylenetetracarboxylates, including mixtures of two or more thereof,
more preferably from the group consisting of optionally protonated malonates, succinates, glutarates, adipates, citrates, isocitrates, aconitates, propane-1,2,3-tricarboxylates, methanetetracarboxylates, and ethylenetetracarboxylates, including mixtures of two or more thereof,
more preferably from the group consisting of optionally protonated succinates, glutarates, citrates, isocitrates, propane-1,2,3-tricarboxylates, and methanetetracarboxylates, including mixtures of two or more thereof,
more preferably from the group consisting of optionally protonated succinates, citrates, isocitrates, and propane-1,2,3-tricarboxylates, including mixtures of two or more thereof,
more preferably from the group consisting of optionally protonated citrates and isocitrates, including mixtures of two or more thereof,
wherein more preferably the one or more complexing agents C comprise optionally protonated citrates, including mixtures of two or more thereof, preferably citrates, including mixtures of two or more thereof
wherein more preferably the one or more complexing agents C consist if optionally protonated citrates, including mixtures of two or more thereof, preferably of citrates, including mixtures of two or more thereof,
wherein the counterion of the unprotonated form or forms of the one or more complexing agents C is selected from the group consisting of alkali metals, alkaline earth metals, ammonium, and combinations of two or more thereof, wherein preferably the counterion is Na⁺ and/or K⁺, preferably Na⁺.

A further preferred embodiment (6) concretizing any one of embodiments (1) to (5) relates to said process, wherein the mixture provided in (a) and heated in (c) or obtained in (b) and heated in (c) does not comprise a complexing agent other than the one or more complexing agents C.

A further preferred embodiment (7) concretizing any one of embodiments (1) to (6) relates to said process, wherein in (b) the pH of the mixture provided in (a) is adjusted to a pH comprised in the range of from 5 to 7, preferably from 5.5 to 6.5, more preferably from 5.9 to 6.3, more preferably from 6.0 to 6.2, and more preferably to about a pH of 6.1.

A further preferred embodiment (8) concretizing any one of embodiments (1) to (7) relates to said process, wherein in (a) the one or more transition metals M are selected from the group consisting of Cu, Ru, Rh, Pd, Ag, Re, Os, Ir, Pt, and Au, including mixtures of two or more thereof, preferably from the group consisting of Pd, Pt, Ru, Rh, Au, and Ag, including mixtures of two or more thereof, more preferably from the group consisting of Pd, Pt, Au, and Ag, including mixtures of two or more thereof, wherein more preferably the one or more transition metals M comprise Pd and/or Pt, preferably Pd, wherein more preferably the one or more transition metals M consist of Pd and/or Pt, preferably of Pd.

A further preferred embodiment (9) concretizing any one of embodiments (1) to (8) relates to said process, wherein in (a) the counterion of the one or more salts of one or more transition metals M is selected from the group consisting of halides, hydroxide, nitrate, phosphate, sulfate, and combinations of two or more thereof, more preferably from the group consisting of chloride, bromide, hydroxide, nitrate, sulfate, and combinations of two or more thereof, wherein more preferably the counterion is chloride and/or nitrate, preferably chloride.

A further preferred embodiment (10) concretizing any one of embodiments (1) to (9) relates to said process, wherein in (a) the one or more salts of the one or more transition metals M are provided as a halide complex, preferably as a chloride complex, and more preferably as a tetrachloro complex, wherein the counterion of the complex is preferably selected from the group consisting of H⁺, alkali metals, alkaline earth metals, ammonium, and combinations of two or more thereof, more preferably from the group consisting of Na⁺, K⁺, H⁺, and combinations of two or more thereof, wherein more preferably the counterion is Na⁺ and/or H⁺, preferably Na⁺.

A further preferred embodiment (11) concretizing any one of embodiments (1) to (10) relates to said process, wherein in the mixture provided in (a), the C : M molar ratio of the one or more complexing agents C to the one or more transition metals M is comprised in the range of from 1 to 200, preferably from 5 to 100, more preferably from 10 to 50, more preferably from 15 to 40, more preferably from 20 to 30, more preferably from 23 to 27, and more preferably from 25 to 26.

A further preferred embodiment (12) concretizing any one of embodiments (1) to (11) relates to said process, wherein the solvent system S employed in (a) comprises one or more polar solvents, preferably one or more polar protic solvents, more preferably one or more polar protic solvents selected from the group consisting of C1-C4 alcohols, water, dimethylformamide, and mixtures of two or more thereof, more preferably from the group consisting of n-propanol, iso-propanol, methanol, ethanol, water, and mixtures of two or more thereof, more preferably from the group consisting of methanol, ethanol, dimethylformamide, water, and mixtures of two or more thereof, wherein more preferably the solvent system comprises ethanol and/or water, preferably water, wherein more preferably the solvent system consists of ethanol and/or water, preferably of water.

A further preferred embodiment (13) concretizing any one of embodiments (1) to (12) relates to said process, wherein in the mixture provided in (a), the S : C molar ratio of the solvent system S to the one or more complexing agents C is comprised in the range of from 1 to 200, preferably from 5 to 100, more preferably from 10 to 50, more preferably from 15 to 40, more preferably from 20 to 30, and more preferably from 24.2 to 26.2.

A further preferred embodiment (14) concretizing any one of embodiments (1) to (13) relates to said process, wherein in the mixture provided in (a), the S : M molar ratio of the solvent system S to the one or more transition metals M is comprised in the range of from 50 to 3500, preferably from 60 to 2000, more preferably from 75 to 1000, more preferably from 300 to 900, preferably from 400 to 800, preferably from 500 to 700, more preferably from 620 to 665, and more preferably from 642 to 643.

A further preferred embodiment (15) concretizing any one of embodiments (1) to (14) relates to said process, wherein in (b) the pH of the mixture provided in (a) is adjusted using a base, preferably using a salt of a hydroxide, more preferably using an alkali metal hydroxide, wherein the alkali metal is preferably selected from the group consisting of Na, K, Rb, and Cs, wherein more preferably the alkali metal hydroxide is sodium hydroxide and/or potassium hydroxide, wherein more preferably the alkali metal hydroxide is sodium hydroxide.

A further preferred embodiment (16) concretizing any one of embodiments (1) to (15) relates to said process, wherein in (b) the pH of the mixture provided in (a) is adjusted using an acid, preferably using a mineral acid, more preferably using a mineral acid selected from the group consisting of HCl, HBr, HI, HClO, HClO₂, HClO₃, HClO₄, H₂SO₄, HSO₃F, HNO₃, H₃PO₄, HSBF₆, HBF₄, and HPF₆, including mixtures of two or more thereof, more preferably from the group consisting of HCl, HBr, HClO₄, H₂SO₄, HSO₃F, HNO₃, and H₃PO₄, including mixtures of two or more thereof, more preferably from the group consisting of HCl, HBr, H₂SO₄, HNO₃, and H₃PO₄, including mixtures of two or more thereof, more preferably from the group consisting of HCl, H₂SO₄, and HNO₃, including mixtures of two or more thereof, wherein more preferably the mixture provided in (a) is adjusted using HCl.

A further preferred embodiment (17) concretizing embodiment (16) relates to said process, wherein the acid is employed as an aqueous solution, preferably as an aqueous solution comprising from 5 to 60 wt.-% of the acid, more preferably from 10 to 50 wt.-%, more preferably from 20 to 40 wt.-%, more preferably from 25 to 39 wt.-%, more preferably from 29 to 37 wt.-%, more preferably from 31 to 35 wt.-%, and more preferably from 32 to 34 wt.-%.

A further preferred embodiment (18) concretizing any one of embodiments (1) to (17) relates to said process, wherein in (c) the mixture provided in (a) or obtained in (b) is heated to a temperature comprised in the range of from 80 to 120 °C, preferably from 90 to 110 °C, more preferably from 95 to 105 °C, more preferably from 98 to 102 °C, and more preferably from 99 to 101 °C.

A further preferred embodiment (19) concretizing any one of embodiments (1) to (18) relates to said process, wherein in (c) the mixture provided in (a) or obtained in (b) is heated to a temperature comprised in the range of from 70 °C to the boiling point of the mixture provided in (a) or obtained in (b), preferably from 90 °C to the boiling point of the mixture provided in (a) or obtained in (b), wherein more preferably the mixture provided in (a) or obtained in (b) is heated to the boiling point of the mixture provided in (a) or obtained in (b), wherein the boiling point refers to the boiling temperature at 100 kPa.

A further preferred embodiment (20) concretizing any one of embodiments (1) to (19) relates to said process, wherein in (c) the mixture provided in (a) or obtained in (b) is heated for a duration comprised in the range of from 1 to 360 minutes, preferably from 5 to 60 minutes, more preferably from 10 to 40 minutes, more preferably from 10 to 20 minutes, more preferably from 12 to 18 minutes, and more preferably about 15 minutes.

A further preferred embodiment (21) concretizing any one of embodiments (1) to (20) relates to said process, wherein in (c) the mixture provided in (a) or obtained in (b) is heated under a pressure comprised in the range of from 90 to 110 kPa, preferably of from 95 to 105 kPa, more preferably of from 98 to 102 kPa, and more preferably from 99 to 101 kPa, wherein more preferably the mixture provided in (a) or obtained in (b) is heated under atmospheric pressure.

A further preferred embodiment (22) concretizing any one of embodiments (1) to (21) relates to said process, wherein the process further comprises:
(e) contacting the colloidal suspension of transition metal nanoparticles obtained in (c) or the transition metal nanoparticles obtained in (d) with a support material for supporting the transition metal nanoparticles on the support material.

A further preferred embodiment (23) concretizing any one of embodiments (1) to (22) relates to said process, wherein the support material in (e) comprises carbon and/or a metal oxide and/or a metalloid oxide, preferably activated carbon and/or an oxide selected from the group consisting of oxides of Si, Al, Ti, Zr, Hf, La, Ce, Pr, Nd, and mixtures and/or mixed oxides of two or more thereof, more preferably from the group consisting of oxides of Si, Al, Ti, Zr, and mixtures and/or mixed oxides of two or more thereof, wherein more preferably the support material is selected from the group consisting of activated carbon, silica, alumina, silica-alumina, aluminosilicates, titanosilicates, and mixtures of two or more thereof, wherein more preferably the support material comprises activated carbon and/or silicalite, preferably activated carbon, wherein more preferably the support material consists of activated carbon and/or silicalite, preferably of activated carbon.

A further preferred embodiment (24) concretizing any one of embodiments (1) to (23) relates to said process, wherein the support material in (e) is a monolith substrate and/or is in the form of granules and/or is in the form of a powder.

A further preferred embodiment (25) concretizing any one of embodiments (1) to (24) relates to said process, wherein contacting in (e) is achieved by impregnation, preferably by incipient wetness impregnation and/or by vacuum impregnation.

An embodiment (26) of the present invention relates to a colloidal suspension of transition metal nanoparticles obtainable and/or obtained according to the process of any of embodiments (1) to (21).

A preferred embodiment (27) concretizing embodiment (26) relates to said colloidal suspension of transition metal nanoparticles, wherein the concentration of the transition metal nanoparticles in the colloidal suspension is comprised in the range of from 0.01 to 5 wt.-%, preferably from 0.1 to 4 wt.-%, more preferably from 0.2 to 3 wt.-%, more preferably from 0.4 to 2 wt.-% and more preferably from 0.5 to 1 wt.-%, wherein the total weight of the transition metal nanoparticles corresponds to the total weight of the transition metal contained in the colloidal suspension calculated as the element.

A further preferred embodiment (28) concretizing embodiment (26) or (27) relates to said colloidal suspension of transition metal nanoparticles, wherein the weight-based average particle size D50 of the transition metal nanoparticles is in the range of from 0.2 to 20 nm, preferably from 0.4 to 10 nm, more preferably from 0.6 to 5 nm, more preferably from 0.8 to 4 nm, more preferably from 1 to 3, nm more preferably from 1.2 to 2.5 nm, more preferably from 1.4 to 2.3 nm, more preferably from 1.6 to 2.1 nm, more preferably from 1.65 to 1.9 nm, and more preferably from 1.7 to 1.8 nm, wherein preferably the particle size distribution is determined according to ISO 13320-1, and the weight-based average particle size D50 is calculated based thereon according to ISO 9276-2:2001.

A further preferred embodiment (29) concretizing any one of embodiments (26) to (28) relates to said colloidal suspension of transition metal nanoparticles, wherein the molecular weight of the transition metal nanoparticles is in the range of from 3000 to 50000 Dalton, preferably in the range of from 4000 to 10000 Dalton, more preferably in the range of from 5000 to 8000 Dalton, preferably determined according to Reference Example 2.

A further preferred embodiment (30) concretizing any one of embodiments (26) to (29) relates to said colloidal suspension of transition metal nanoparticles, wherein the transition metal of the nanoparticles is selected from the group consisting of Cu, Ru, Rh, Pd, Ag, Re, Os, Ir, Pt, Au, and alloys of two or more thereof, preferably from the group consisting of Pd, Pt, Ru, Rh, Au, Ag, and alloys of two or more thereof, and more preferably from the group consisting of Pd, Pt, Au, Ag, and alloys of two or more thereof, wherein more preferably the transition metal of the nanoparticles comprises Pd, Pt, PdAu, PdAg, PtAu, or PtAg, preferably Pd, PdAg, or PdAu, more preferably Pd or PdAu, and more preferably Pd, wherein more preferably the transition metal of the nanoparticles is Pd, Pt, PdAu, PdAg, PtAu, or PtAg, preferably Pd, PdAg, or PdAu, more preferably Pd or PdAu, and more preferably Pd.

An embodiment (31) of the present invention relates to transition metal nanoparticles obtainable and/or obtained according to the process of any one of embodiments (1) to (21), in particular according to any one of embodiments (1) to (21) for the process for the preparation of transition metal nanoparticles comprising (d).

A further preferred embodiment (32) concretizing embodiment (31) relates to said transition metal nanoparticles, wherein the weight-based average particle size D50 of the transition metal nanoparticles is in the range of from 0.2 to 20 nm, preferably from 0.4 to 10 nm, more preferably from 0.6 to 5 nm, more preferably from 0.8 to 4 nm, more preferably from 1 to 3, nm more preferably from 1.2 to 2.5 nm, more preferably from 1.4 to 2.3 nm, more preferably from 1.6 to 2.1 nm, more preferably from 1.65 to 1.9 nm, and more preferably from 1.7 to 1.8 nm, wherein preferably the particle size distribution is determined according to ISO 13320-1, and the weight-based average particle size D50 is calculated based thereon according to ISO 9276-2:2001.

A further preferred embodiment (33) concretizing embodiment (31) or (32) relates to said transition metal nanoparticles, wherein the molecular weight of the transition metal nanoparticles is in the range of from 3000 to 50000 Dalton, preferably in the range of from 4000 to 10000 Dalton, more preferably in the range of from 5000 to 8000 Dalton, preferably determined according to Reference Example 2.

A further preferred embodiment (34) concretizing any one of embodiments (31) to (33) relates to said transition metal nanoparticles, wherein the transition metal of the nanoparticles is selected from the group consisting of Cu, Ru, Rh, Pd, Ag, Re, Os, Ir, Pt, Au, and alloys of two or more thereof, preferably from the group consisting of Pd, Pt, Ru, Rh, Au, Ag, and alloys of two or more thereof, and more preferably from the group consisting of Pd, Pt, Au, Ag, and alloys of two or more thereof, wherein more preferably the transition metal of the nanoparticles comprises Pd, Pt, PdAu, PdAg, PtAu, or PtAg, preferably Pd, PdAg, or PdAu, more preferably Pd or PdAu, and more preferably Pd, wherein more preferably the transition metal of the nanoparticles is Pd, Pt, PdAu, PdAg, PtAu, or PtAg, preferably Pd, PdAg, or PdAu, more preferably Pd or PdAu, and more preferably Pd.

An embodiment (35) of the present invention relates to a catalyst comprising transition metal nanoparticles obtainable and/or obtained according to the process of any one of embodiments (22) to (25).

A preferred embodiment (36) concretizing embodiment (35) relates to said catalyst, wherein the catalyst comprises from 0.01 to 10 wt.-% of transition metal nanoparticles calculated as the transition metal and based on 100 wt.-% of the support material, preferably from 0.05 to 6 wt.-%, more preferably from 0.1 to 4 wt.-%, more preferably from 0.3 to 3 wt.-%, more preferably from 0.5 to 2.5 wt.-%, more preferably from 0.6 to 2 wt.-%, more preferably from 0.7 to 1.5 wt.-%, more preferably from 0.8 to 1.3 wt.-%, and more preferably from 0.9 to 1.1 wt.-%.

An embodiment (37) of the present invention relates to a use of a catalyst according to embodiment (35) or (36) as a hydrogenation catalyst, preferably as a hydrogenation catalyst in the production of hydrogen peroxide, and more preferably as a hydrogenation catalyst in the anthraquinone process for the production of hydrogen peroxide.

### EXPERIMENTAL SECTION

### Reference Example 1: TEM and determination of particle size distribution and of average particle size

The particle size distribution and the average particle size was determined by analyzing the samples obtained according to the examples using a PHILIPS EM208S (FEI) transmission electronic microscope equipped with a MEGA VIEW III (OLYMPUS) acquisition camera, wherein the data was analyzed and processed using the IMAGE J software package.

### Reference Example 2: Determination of molecular weight

The determination of molecular weight was carried out by ultrafiltration with two different semipermeable membranes: 3000 NMWL (nominal molecular weight limit) and 50000 NMWL (nominal molecular weight limit), using a Sorvall ST 16 Centrifuge unit.

Approximately 1 - 2 mL of a sample solution was transferred to an ultrafiltration tube and centrifuged at 5000 rpm for 10 minutes.

The content of Palladium in the filtrate, after separation trough the membrane, was determined by analysis by ICP-OES.

### Example 1: Preparation of colloidal Pd

In a 100 ml flask were added 50 g of H₂O and 35.3 g of Na₃Ct · H₂O (trisodium citrate dehydrate; 120 mmol). The resulting mixture was stirred until complete dissolution of the citrate. Then 2.76 g of Na₂PdCl₄ (corresponding to 0.5 g Pd; 4.7 mmol Pd) were added. The molar ratio of Pd : Na₃Ct was 1 : 25.5. The pH(25 °C) of the resulting mixture was determined as being 6.52. Then, the pH(25 °C) was lowered to 6.1 with aqueous HCl 33 % (33 weight-% of HCl in deionized water). The color of the solution was red. Then, the solution was heated until the boiling point of 100 °C. After 15 minutes of heating, the reaction was stopped. During that time the color of the solution changed to brown.

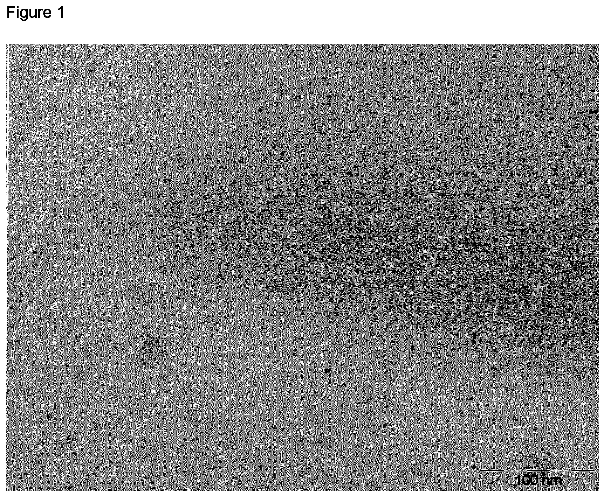

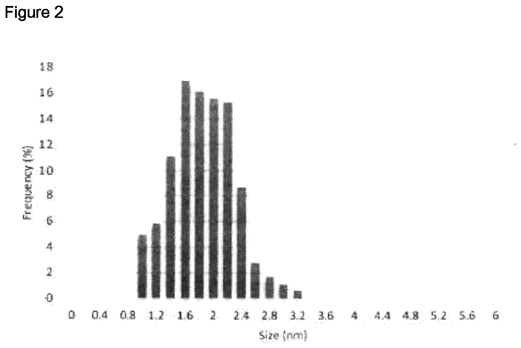

The resulting product was analyzed with an ultracentrifugation apparatus. The molecular weight of the resulting nanoparticles was determined as being between 3000 and 50000 Dalton. The average particle size of the resulting nanoparticles was determined as being 1.75 ± 0.44 nm. The TEM image of the nanoparticles is shown in Figure 1. The particle size distribution of the colloidal Pd of Example 1 is shown in Figure 2.

### Brief description of figures

Figure 1: shows a TEM image of a sample colloidal suspension prepared according to Example 1.
Figure 2: shows a particle size distribution of a sample colloidal suspension prepared according to Example 1. On the abscissa, the particle size is given in nm and on the ordinate the frequency is given in %.

### Cited literature

- M. Michaelis et al. in J. Phys. Chem. 1994, 98, pp. 6212-6215
- M. Shao et al. in Chem. Commun. 2011, 47, pp. 6566-6568
- Z.-L. Wang et al. in Scientific Reports 2 : 598, pp. 1-6
- H. Lv et al. in Nano Energy 2016, 29, 149-165
- US 10,493,433 B2

## Claims

1. A process for the preparation of transition metal nanoparticles, the process comprising:
(a) providing a mixture of one or more salts of one or more transition metals M, one or more complexing agents C, and a solvent system S;
(b) optionally adjusting the pH of the mixture provided in (a) to a pH comprised in the range of from 4 to 8;
(c) heating the mixture provided in (a) or obtained in (b) for obtaining a colloidal suspension of transition metal nanoparticles; and
(d) optionally isolating the transition metal nanoparticles obtained in (c), preferably by centrifugation and/or evaporation to dryness of the colloidal suspension obtained in (c); wherein the mixture provided in (a) and heated in (c) or obtained in (b) and heated in (c) does not comprise polyvinyl sulfate and/or polyvinylpyrrolidone.

2. The process of claim 1, wherein the mixture provided in (a) and heated in (c) or obtained in (b) and heated in (c) does not comprise a polyvinyl sulfate, polyvinylpyrrolidone, a copolymer comprising vinylpyrrolidone, and/or a fatty acid-substituted or unsubstituted polyoxyethylene.

3. The process of claim 1 or 2, wherein the mixture provided in (a) and heated in (c) or obtained in (b) and heated in (c) does not comprise ascorbic acid, an ascorbate, formic acid, a formate, and/or a borohydride.

4. The process of any of claims 1 to 3, wherein the mixture provided in (a) and heated in (c) or obtained in (b) and heated in (c) consists of the mixture of the one or more salts of one or more transition metals M, the one or more complexing agents C, the solvent system, and optionally one or more compounds employed for adjusting the pH of the mixture in (b).

5. The process of any of claims 1 to 4, wherein the one or more complexing agents C are selected from the group consisting of carboxylates and salts thereof.

6. The process of any of claims 1 to 5, wherein the mixture provided in (a) and heated in (c) or obtained in (b) and heated in (c) does not comprise a complexing agent other than the one or more complexing agents C.

7. The process of any of claims 1 to 6, wherein in (b) the pH of the mixture provided in (a) is adjusted to a pH comprised in the range of from 5 to 7.

8. The process of any of claims 1 to 7, wherein in (a) the one or more transition metals M are selected from the group consisting of Cu, Ru, Rh, Pd, Ag, Re, Os, Ir, Pt, and Au, including mixtures of two or more thereof.

9. The process of any of claims 1 to 8, wherein in the mixture provided in (a), the C : M molar ratio of the one or more complexing agents C to the one or more transition metals M is comprised in the range of from 1 to 200.

10. The process of any of claims 1 to 9, wherein in the mixture provided in (a), the S : M molar ratio of the solvent system S to the one or more transition metals M is comprised in the range of from 50 to 3500.

11. The process of any of claims 1 to 10, wherein the process further comprises:
(e) contacting the colloidal suspension of transition metal nanoparticles obtained in (c) or the transition metal nanoparticles obtained in (d) with a support material for supporting the transition metal nanoparticles on the support material.

12. A colloidal suspension of transition metal nanoparticles obtainable and/or obtained according to the process of any one of claims 1 to 10.

13. Transition metal nanoparticles obtainable and/or obtained according to the process of any one of claims 1 to 10, preferably according to any one of claims 1 to 10 comprising (d).

14. A catalyst comprising transition metal nanoparticles obtainable and/or obtained according to the process of claim 11.

15. Use of a catalyst according to claim 14 as a hydrogenation catalyst.
